# EUROPEAN PATENT APPLICATION

(11) **EP 2 468 254 A1**
(43) Date of publication of application: **27.06.2012**
(21) Application number: 10195186.1
(22) Date of filing: 15.12.2010
(51) Int. Cl.: A61K 9/00, A61K 9/20, A61K 31/445

(54) **Orally disintegrating tablet having a taste masking effect**

(71) Applicant: Hexal AG, 83607 Holzkirchen (DE)
(72) Inventor: Otto, Ina, 83607 Holzkirchen (DE)
(74) Representative: Henkel, Breuer & Partner

(57) **Abstract**

The present invention is directed to an orally disintegrating tablet having a taste masking effect, comprising at least one unpleasant-tasting pharmaceutically active ingredient having a particle size d(0.1) of 50 µm or more.

## Description

### Field of the Invention

The present invention relates to orally disintegrating tablets comprising at least one unpleasant-tasting, in particular bitter-tasting, pharmaceutically active ingredient wherein the unpleasant taste is efficiently masked.

### Background of the Invention

Orally disintegrating tablets, also called orodispersible tablets, are a drug dosage form, designed to be dissolved on the tongue of a patient, rather than swallowed as a whole. Orally disintegrating tablets quickly disintegrate in the mouth without requiring water. They are an advantageous dosage form for patients who have difficulties in swallowing, in particular old people and infants, and for patients where compliance is a known issue and therefore an easier dosage form ensures that the medication is in fact taken. Additionally, since orally disintegrating tablets can be taken without water, they are convenient in situations where water is not easily available.

A large variety of drugs possess an unpleasant taste. In particular, a bitter taste often occurs. If such unpleasant-tasting drugs are to be administered in the form of an orally disintegrating tablet, their taste has to be masked. Otherwise, the patient's compliance would be impaired due to the nature of orally disintegrating tablets to quickly dissolve in the mouth and release the unpleasant-tasting drug into the oral cavity where it can stimulate the taste buds on the surface of the tongue in an undesired manner.

Several approaches for taste masking in pharmaceuticals are known, such as coating, microencapsulation, addition of sweeteners or other flavouring agents, taste suppressants or complex formation.

Presumably the most common method for taste masking, in particular for masking a bitter taste, is to use a flavouring agent, such as a sweetener. While this is often effective for mildly bitter compounds, with highly bitter compounds a heavy, unpleasant aftertaste is often experienced despite the flavouring agent.

EP 0 974 366 discloses the use of an anionic polysaccharide, such as carrageenan, chondroitin sulfate, dextran sulfate, alginic acid, gellan gum, xanthan gum or salts thereof, to mask the unpleasant taste of a drug. The examples show the use of an anionic polysaccharide, in a ratio of at least 1:1 with the bitter tasting medicine, in the manufacture of granules, jellies and syrups. While a masking effect of the bitter medicine is shown, the quantity of the anionic polysaccharide required to achieve this effect is relatively high.

One of the present inventors recently discovered that zinc salts are effective in masking a bitter taste of a drug, as described in WO 2010/119009.

However, each of the above approaches makes use of at least one taste-masking agent. As a consequence, each of these techniques require the inclusion of one or more additional excipients, usually involving one or more additional process steps during manufacturing. Needless to say that this leads to increased manufacturing costs. Moreover, there is a risk that these taste masking agents impair the stability of the drug dosage form, for instance by interaction or chemical reaction with the drug or the further excipients, in particular during long-term storage. Furthermore, since every pharmaceutical product has to be admitted by regulatory authorities, such as the EMA (formerly known as EMEA) in the European Union or the FDA in the US, the taste masking agents also have to pass admittance. Finally, there is a risk that a non-homogenous mixing of the unpleasant-tasting drug and the taste masking agent can lead to localized areas of unpleasant taste in the mouth when ingested.

### Object of the Invention

The object of the present invention is to provide orally disintegrating tablets comprising at least one unpleasant-tasting, in particular bitter-tasting, pharmaceutically active ingredient wherein the unpleasant taste is efficiently masked without the need of a taste masking agent.

### Detailed Description of the Invention

The present inventors have now surprisingly found that a taste masking effect can be achieved by using an unpleasant-tasting pharmaceutically active ingredient having a sufficiently large particle size when formulated in an orally disintegrating tablet.

Accordingly, the present invention relates to an orally disintegrating tablet, comprising at least one unpleasant-tasting pharmaceutically active ingredient having a particle size d(0.1) of 50 µm or more.

In preferred embodiments of the present invention, the particle size d(0.1) of the at least one unpleasant-tasting pharmaceutically active ingredient is more than 50 µm, more preferably 70 µm or more, still more preferably more than 70 µm.

The at least one unpleasant-tasting pharmaceutically active ingredient according to the present invention has preferably an average particle size d(0.5) of 100 µm or more, more preferably more than 100 µm.

The term "particle size d(0.1)", as used herein, denotes the value of the particle size up to which cumulatively 10% of the particles in the particle size distribution were found to lie. Accordingly, the particle size d(0.1) corresponds to the 0.1-quantile of the particle size distribution. In other words, a particle size d(0.1) of 50 µm or more denotes that 90% of the particles have a particle size of 50 µm or more.

The term "average particle size d(0.5)", as used herein, denotes the value of the particle size up to which cumulatively 50% of the particles in the particle size distribution were found to lie. Accordingly, the average particle size d(0.5) corresponds to the median of the particle size distribution. In other words, an average particle size d(0.5) of 100 µm or more denotes that 50% of the particles have a particle size of 100 µm or more.

The determination of the particle size d(0.1), the average particle size d(0.5) as well as the particle size distribution is within the general knowledge of a person skilled in the art. Suitable methods include laser diffraction, as described for instance in European Pharmacopoeia 6.1, Chapter 2.9.31, and the method described below using a Malvern Mastersizer 2000.

The term "pharmaceutically active ingredient", also referred to herein as "drug", denotes a compound having a pharmacological effect in the human or animal body and is in particular suitable for treating, alleviating or preventing diseases or other undesired conditions in a human or animal body.

The term "unpleasant-tasting pharmaceutically active ingredient" denotes a pharmaceutically active ingredient which exerts an unpleasant taste when present in a human or animal oral cavity.

The expression "comprising", as used herein, includes not only the meaning of "comprising" but also encompasses "consisting essentially of" and "consisting of".

Without wishing to be bound by any theory, the present inventors assume that the taste masking effect according to the present invention is due to an incomplete dissolution of the large sized particles of the unpleasant-tasting drug in the mouth prior to swallowing. As a consequence, the surface of the drug particles, which might come into contact with the surface of the tongue, thereby stimulating the taste buds in an undesired manner, is significantly decreased, compared with conventional drug particles having a relatively small particle size. Thereby, the unpleasant-tasting drug particles according to the present invention do not exert their full unpleasant taste before being swallowed. Conventional drug particles usually have a comparatively small particle size, in particular due to pharmaceutical and pharmacological reasons and owing to their manufacturing process involving grinding and abrasion. However, in an orally disintegrating tablet, it is not necessary for the pharmaceutically active ingredient to be dissolved entirely in the oral cavity because absorption into the patient's body is intended to be enterally, rather than buccally.

The unpleasant-tasting pharmaceutically active ingredient according to the present invention is preferably a bitter-tasting pharmaceutically active ingredient.

The unpleasant-tasting pharmaceutically active ingredient or the bitter-tasting pharmaceutically active ingredient according to the present invention preferably has a solubility in water at 20 °C of 30 mg/ml or more, more preferably more than 30 mg/ml, still more preferably 50 mg/ml or more, still more preferably more than 50 mg/ml. Despite its good water solubility, such unpleasant-tasting or bitter-tasting pharmaceutically active ingredients can be administered in an orally disintegrating tablet according to the present invention.

Examples of pharmaceutically active ingredients according to the present invention include donepezil hydrochloride, dextromethorphan, chlorhexidine, guaifenesin, pseudoephedrine, caffeine, atorvastatin, acetyl salicylic acid, acetaminophen, diphenhydramine, doxylamine, sildenafil citrate, loperamide, midodrine hydrochloride, brotizolam, ticlopidine hydrochloride, maprotiline hydrochloride, ifenprodil tartrate, berberine hydrochloride, digitoxin, sulpyrine, azelastine hydrochloride, etilefrine hydrochloride, diltiazen hydrochloride, propranolol hydrochloride, chloramphenicol, aminophylline, erythromycin, phenobarbital, calcium pantothenate or pantothenic acid, indeloxazine hydrochloride, aminoguanidine hydrochloride, ibuprofen and cefcapene hydrochloride.

In a preferred embodiment of the present invention, the bitter-tasting pharmaceutically active ingredient is donepezil (1-benzyl-4-[(5,6-dimethoxyindan-1-on-2-yl)methyl]piperidine), more preferably donepezil hydrochloride. Donepezil hydrochloride is centrally acting as a reversible acetylcholine esterase inhibitor and is used for the treatment of Alzheimer's disease of a slight to medium degree. Donepezil hydrochloride has a good water solubility and its aqueous solution has a sharp bitterness. Typically, donepezil hydrochloride is available on the market with average particle sizes of approximately 9 to 15 µm. The present inventors surprisingly found that, despite its good water solubility and high bitterness, donepezil hydrochloride can be administered in an orally disintegrating tablet according to the present invention wherein the donepezil hydrochloride has a particle size d(0.1) of 50 µm or more without requiring a taste masking agent.

The orally disintegrating tablet according to the present invention preferably comprises at least one disintegrant.

Any disintegrant customary in the pharmaceutical sector may be used as a disintegrant. Examples thereof include starches and starch derivatives, in particular sodium starch glycolate, polyvinylpyrrolidone, in particular cross-linked polyvinylpyrrolidone, carboxymethylcellulose sodium or croscarmellose sodium, in particular low-substituted carboxymethylcellulose sodium, and mixtures thereof.

Preferably, the at least one disintegrant according to the present invention comprises sodium starch glycolate.

The orally disintegrating tablet according to the present invention preferably comprises at least one filler.

Any filler customary in the pharmaceutical sector may be used as a filler. Examples thereof include sugars and sugar alcohols, such as lactose, sucrose, glucose and mannitol, macromolecular fillers, such as microcrystalline cellulose, starches, in particular maize starch, rice starch, potato starch and wheat starch, and mixtures thereof.

Preferably, the at least one filler according to the present invention comprises mannitol because mannitol exerts a good and smooth feeling in the mouth.

According to the present invention, the masking of an unpleasant taste is efficiently achieved without the need for a taste masking agent.

In a preferred embodiment of the present invention, the orally disintegrating tablet does not comprise a taste masking agent. In particular, it is preferred that the orally disintegrating tablet does not comprise an anionic polysaccharide, such as carrageenan, chondroitin sulfate, dextran sulfate, alginic acid, gellan gum, xanthan gum or salts thereof.

However, it is of course within the scope of the present invention that the tablet may also comprise one or more taste masking agents. Preferred taste masking agents according to the present invention are flavouring agents, sweeteners and zinc salts.

While flavouring agents and sweeteners are conventionally not sufficiently effective with highly bitter compounds, such as donepezil hydrochloride, they are suitable in the present invention for masking a possibly remaining slight unpleasant or bitter taste, depending on the bitterness and the amount of the pharmaceutically active ingredient used. Suitable sweeteners include cyclamate, saccharin, acesulfame, aspartame, sucralose, thaumatin, neohesperidine, and mixtures thereof, while other sweeteners may also be suitable. Suitable flavouring agents include lemon, orange, grapefruit, strawberry, cherry, peppermint, menthol, and mixtures thereof, while other flavouring agents are of course also suitable.

Likewise, it might be advantageous to add a zinc salt in order to suppress any remaining slightly bitter taste and to reduce a numbness occurring when the active ingredient contacts the mucosa. Suitable zinc salts include zinc chloride, zinc bromide, zinc iodide, zinc sulfate and zinc acetate, and mixtures thereof, while other zinc salts may also be suitable.

The orally disintegrating tablet according to the present invention may comprise additional excipients well known in the art including for instance binders, lubricants, flow regulators, anti-sticking agents and pigments.

Examples of suitable binders include starches, such as maize starch, rice starch, potato starch and wheat starch, gelatine, tragacanth, cellulose ethers, such as hydroxypropylcellulose and hydroxypropylmethylcellulose, polyvinylpyrrolidones, also referred to as polyvidones or povidones, in particular copovidones, and mixtures thereof. Suitable lubricants include fatty acids, in particular stearic acid, salts of fatty acids with divalent metals, in particular magnesium stearate, fatty alcohols, in particular stearyl alcohol, polyethylene glycols, talc, and mixtures thereof. Suitable examples of a flow regulator include colloidal silica. Suitable examples of an anti-sticking agent include talc. Suitable examples of a pigment include titanium oxide.

The method of manufacturing the orally disintegrating tablet according to the present invention is not particularly limited and any method customary to the manufacturing of orally disintegrating tablet can be used. Preference is given to a direct compression, preferably at low forces, in particular a loose compression tabletting. However, also a lyophilisation process represents a suitable method for manufacturing the orally disintegrating tablet according to the present invention.

The amount of the unpleasant-tasting pharmaceutically active ingredient in the orally disintegrating tablet according to the present invention is governed by the desired pharmacological effects of the respective drug and furthermore depends in particular on the age, sex and weight of the patient. In the case of donepezil hydrochloride, typical amounts thereof in the orally disintegrating tablet according to the present invention are for example 5 mg and 10 mg, based on donepezil respectively.

Exemplary compositions of an orally disintegrating tablet according to the present invention are as follows:
- 5 mg donepezil hydrochloride
- 75 mg mannitol
- 45 mg microcrystalline cellulose
- 3 mg aspartame
- 5 mg sodium starch glycolate
- 2 mg magnesium stearate

In the above exemplary compositions, different sorts of donepezil hydrochloride are used, having a particle size d(0.1) of 50 µm and 70 µm, respectively.

Particle size determination using a Malvern Mastersizer 2000:
Preparation of the test sample:
   About 100 mg substance is dispersed in 7 ml silicone oil by sonification for 5 minutes (in test tube or beaker). The sample is measured immediately after the preparation. When removing the dispersed sample with a pipette, care must be taken that no sedimentation has taken place. In either the beaker, test tube or pipette, sedimentation is best prevented by e.g. placing it on a vortex or by charging and discharging the pipette.
Measurement:
   Fractional samples of the test preparation are dropped into the measuring cell containing 80 ml silicone oil until the correct obscuration (10-25%) is reached and then measured. The measurement is performed under stirring at 1800 rpm.

## Claims

1. An orally disintegrating tablet, comprising at least one unpleasant-tasting pharmaceutically active ingredient having a particle size d(0.1) of 50 µm or more.

2. The orally disintegrating tablet according to claim 1, wherein the particle size d(0.1) of said at least one unpleasant-tasting pharmaceutically active ingredient is 70 µm or more.

3. The orally disintegrating tablet according to claim 1 or 2, wherein said at least one unpleasant-tasting pharmaceutically active ingredient has an average particle size d(0.5) of 100 µm or more.

4. The orally disintegrating tablet according to any one of the preceding claims, wherein the at least one unpleasant-tasting pharmaceutically active ingredient is a bitter-tasting pharmaceutically active ingredient.

5. The orally disintegrating tablet according to any one of the preceding claims, wherein the at least one unpleasant-tasting pharmaceutically active ingredient has a solubility in water at 20 °C of 30 mg/ml or more.

6. The orally disintegrating tablet according to claim 4 or 5, wherein the bitter-tasting pharmaceutically active ingredient is donepezil hydrochloride.

7. The orally disintegrating tablet according to any one of the preceding claims, wherein the tablet further comprises at least one disintegrant.

8. The orally disintegrating tablet according to claim 7, wherein said at least one disintegrant comprises sodium starch glycolate.

9. The orally disintegrating tablet according to any one of the preceding claims, wherein the tablet further comprises at least one filler.

10. The orally disintegrating tablet according to claim 9, wherein said at least one filler comprises mannitol.

11. The orally disintegrating tablet according to any one of the preceding claims, wherein the tablet does not comprise a taste masking agent.

12. The orally disintegrating tablet according to any one of the preceding claims, wherein the tablet does not comprise an anionic polysaccharide, selected from the group consisting of carrageenan, chondroitin sulfate, dextran sulfate, alginic acid, gellan gum, xanthan gum and salts thereof.

13. The orally disintegrating tablet according to any one of claims 1 to 10, wherein the tablet further comprises at least one taste masking agent, selected from the group consisting of flavouring agents, sweeteners and zinc salts.
